# FASCICULE DE BREVET EUROPEEN

(11) **EP 1 899 117 B1**
(45) Date de publication et mention de la délivrance du brevet: **01.07.2015**
(21) Numéro de dépôt: 06778612.9
(22) Date de dépôt: 19.06.2006
(51) Int. Cl.: B26B 19/02, B26B 19/06, B26B 19/28, B26B 19/48, A45D 26/00, A61H 7/00, A61B 17/54

(54) **APPAREIL DE TYPE RASOIR, TONDEUSE, EPILATEUR ET/OU "EXFOLIATEUR", À PLUSIEURS TÊTES AMOVIBLES**
MIT MEHREREN ENTFERNBAREN KÖPFEN VERSEHENE RASIER-, SCHNEID-, EPILIER- UND/ODER EXFOLIATIONSVORRICHTUNG
RAZOR, TRIMMER, EPILATOR AND/OR EXFOLIATOR DEVICE PROVIDED WITH A PLURALITY OF REMOVABLE HEADS

(30) Priorité: 20.06.2005 FR 0506200
(43) Date de publication de la demande: 19.03.2008
(73) Titulaire: SEB S.A., 69130 Ecully (FR)
(72) Inventeur: ROZET, Gilbert, F-69003 Lyon (FR)
(74) Mandataire: Guéry-Jacques, Géraldine
(86) Numéro de dépôt international: PCT/FR2006/001377
(87) Numéro de publication internationale: WO 2006/136685

(56) Documents cités:
- EP-A1- 0 630 596
- WO-A2-03/079845
- CH-A5- 662 302

## Description

La présente invention concerne un appareil de type rasoir, tondeuse, épilateur et/ou exfoliateur, à au moins une tête amovible.

Il est connu d'utiliser des appareils distincts pour réaliser un rasage, une tonte, une épilation ou une "exfoliation" (c'est-à-dire une abrasion légère de la peau pour éliminer les peaux mortes), chaque appareil comprenant une tête non amovible et un mécanisme de transmission propre entre le moteur et la tête.

Ces différents appareils ont pour inconvénient, lorsqu'on les possède tous, d'être relativement encombrants et coûteux.

Il est connu du document CH662302 un appareil de soins du corps muni de différents outils démontables du boîtier.

Il est également connu de réaliser un appareil comprenant un corps sur lequel peuvent être adaptées différentes têtes, le corps incluant le moteur d'entraînement et un arbre de sortie unique, et les différentes têtes incluant les différents mécanismes de transmission et/ou de transformation de mouvement adaptés aux différents types de mouvements à réaliser.

Ces appareils ont pour inconvénient d'obliger à prévoir dans chaque tête un système de transmission propre à chaque mouvement à obtenir. Les têtes ont par conséquent des structures relativement complexes et onéreuses à fabriquer.

L'objectif de la présente invention est de remédier à cet inconvénient essentiel.

L'appareil qu'elle concerne comprend, de manière connue en soi, un corps d'appareil incluant un moteur et une transmission, et plusieurs têtes amovibles adaptables sur ce corps, au moins une des têtes amovibles comprenant un sous-ensemble mobile selon un mouvement de translation alternatif et au moins une autre des têtes amovibles comprenant un sous-ensemble mobile selon un mouvement de rotation.

Conformément à l'invention,
- la transmission comprend un organe de transformation de mouvement, entraîné en rotation par l'arbre de sortie du moteur ou par un pignon que comprend la transmission, le sous-ensemble mobile selon un mouvement de translation alternatif comprenant un moyen d'accouplement pouvant être accouplé avec ledit organe de transformation de mouvement lors de l'adaptation de la tête incluant ce sous-ensemble sur ledit corps d'appareil, et
   pouvant être désaccouplé d'avec cet organe de transformation de mouvement lors du retrait de cette tête par rapport audit corps d'appareil ; et
- la transmission comprend un organe de transmission de mouvement de rotation, indépendant dudit organe de transformation de mouvement, le sous-ensemble mobile selon un mouvement de rotation comprenant un moyen d'accouplement pouvant être accouplé avec ledit organe de transmission de mouvement de rotation lors de l'adaptation de la tête incluant ce sous-ensemble sur le corps de l'appareil, et pouvant être désaccouplé d'avec cet organe de transmission lors du retrait de cette tête par rapport audit corps d'appareil.

L'appareil selon l'invention comprend ainsi une transmission incluant un organe de transformation de mouvement, d'une part, et un organe de transmission de mouvement de rotation, d'autre part, indépendants l'un de l'autre ; les têtes amovibles comprennent des moyens d'accouplement respectifs, venant en prise avec l'un ou l'autre de ces organes. Ces têtes n'incluent ainsi aucun organe de transformation de mouvement en elles et ont par conséquent des structures relativement simples.

L'appareil comprend au moins une tête incluant un sous-ensemble mobile selon un mouvement de translation alternatif. Cette tête est en particulier un rasoir, une tondeuse ou un "exfoliateur" (c'est-à-dire réalisant une abrasion légère de la peau pour éliminer les peaux mortes).

L'appareil comprend au moins une tête incluant un sous-ensemble mobile selon un mouvement de rotation. Cette tête est en particulier un épilateur.

Selon une forme de réalisation préférée de l'invention,
- ledit organe de transformation de mouvement est formé par une came de forme cylindrique, comprenant une gorge inclinée par rapport à l'axe de révolution de cette came ;
- ledit moyen d'accouplement du sous-ensemble mobile selon un mouvement de translation alternatif est formé par une patte solidaire du sous-ensemble mobile et comprenant une partie d'extrémité libre destinée à être engagée dans la gorge de ladite came lors de l'adaptation de la tête sur le corps d'appareil.

Ledit organe de transmission de mouvement de rotation peut être quant à lui formé par un pignon faisant saillie du corps d'appareil.

Ladite partie d'extrémité libre de ladite patte présente avantageusement une forme ogival, c'est-à-dire une extrémité libre pointue et des bords latéraux convexes.

Cette forme ogivale permet, grâce à la pointe qu'elle présente, une insertion de ladite partie d'extrémité libre dans la gorge de la came quelles que soient la position angulaire de la came et la position en translation de la partie d'extrémité libre, et permet ainsi d'assurer l'accouplement de cette partie d'extrémité libre et de cette came quelles que soient les positions relatives de cette partie d'extrémité libre et de cette came.

Lesdits bords latéraux convexes permettent de limiter les frottements de la partie d'extrémité libre avec les flancs de la came délimitant latéralement la gorge.

La came peut notamment former corps avec le pignon qui permet l'entraînement de cette came en rotation. Elle peut être montée sur le corps de l'appareil au moyen d'un axe chassé dans des paliers que comprend le corps d'appareil et au travers de l'ensemble formé par cette came et ce pignon.

L'invention sera bien comprise, et d'autres caractéristiques et avantages de celle-ci apparaîtront, en référence au dessin schématique annexé, représentant, à titre d'exemple non limitatif, une forme de réalisation préférée de l'appareil qu'elle concerne.

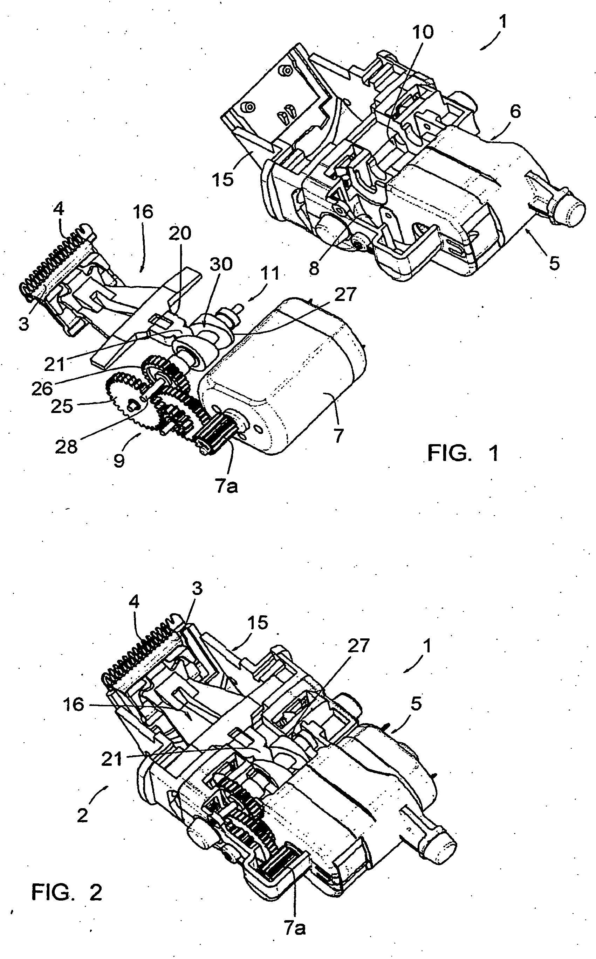

La figure 1 est une vue en perspective éclatée de différents organes et sous-ensembles que comprend cet appareil ;
la figure 2 est une vue des organes et sous-ensembles montrés sur la figure 1, après montage ;
la figure 3 est une vue en perspective éclatée, sous un autre angle, de différents organes et sous-ensembles que comprend cet appareil, et
la figure 4 et une vue de ces organes et sous-ensembles après montage, en coupe passant par l'axe d'une came que comprend.cet appareil.

Les figures représentent, sous différents angles, différents organes et sous-ensembles d'un appareil de type rasoir, tondeuse ou épilateur, à têtes amovibles.

L'appareil comprend un corps d'appareil 1 et plusieurs têtes amovibles 2 adaptables sur ce corps 1. Sur les figures, une seule tête 2 est représentée, à savoir une tête de tondeuse comprenant une lame 3 mobile selon un mouvement de translation alternatif et une contre-lame fixe 4, montée sur la tête 2. L'appareil peut comprendre au moins une autre tête 2 à mouvement de translation alternatif, formant un rasoir ou un exfoliateur (c'est-à-dire réalisant une abrasion légère de la peau pour éliminer les peaux mortes), et au moins une autre tête 2 comprenant un sous-ensemble mobile selon un mouvement de rotation, en particulier un épilateur.

Le corps d'appareil 1 comprend un châssis 5 placé dans un boîtier (non représenté), ce châssis 5 formant notamment un logement 6 de réception d'un moteur 7, un logement 8 de réception d'un train de pignons 9 et un logement 10 de réception d'un ensemble 11 formé par une came et un pignon engagés sur un axe.

Le châssis 5 comprend en outre des moyens pour le montage amovible de chaque tête 2, ne faisant pas particulièrement partie de la présente invention, et donc non particulièrement détaillés.

Chaque tête 2 comprend un châssis 15 destiné à être placé dans un boîtier (non représenté), comportant la contre-lame 4 et logeant un sous-ensemble 16 mobile par rapport à lui selon un mouvement de translation alternatif. Le châssis 15 forme des moyens de guidage du mouvement du sous-ensemble 16, et ce sous-ensemble 16 comporte la lame 3 et des moyens de guidage complémentaires de ceux du châssis 15.

Le sous-ensemble 16 comprend une patte médiane saillante 20 dont la partie d'extrémité libre 21 présente une forme ogivale, c'est-à-dire comprend une extrémité libre pointue et des bords latéraux convexes.

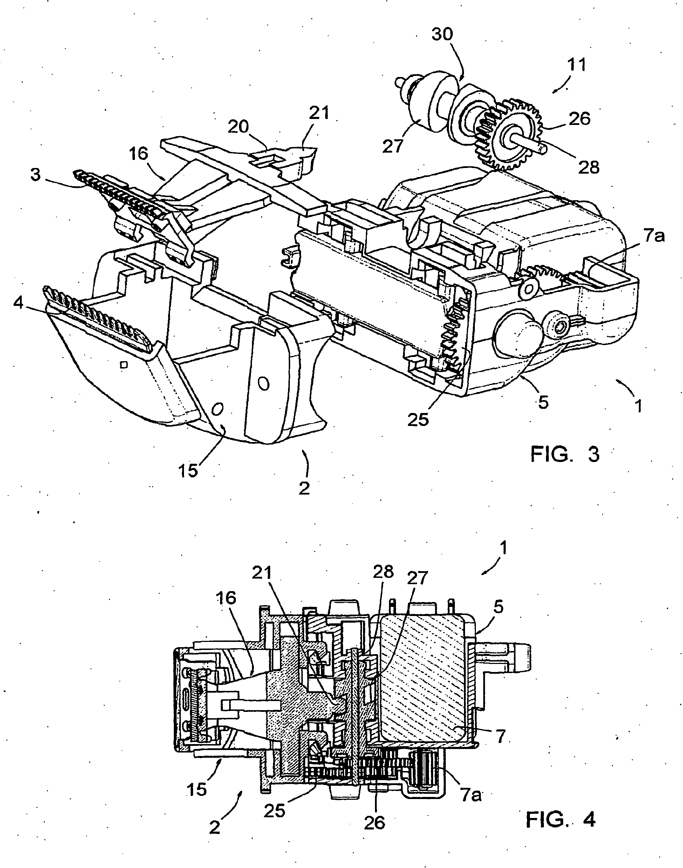

Le moteur 7 comprend un pignon de sortie 7a dépassant, après montage, du logement 6 du châssis 5 et engrenant avec le pignon central d'une roue dentée triple. Un autre pignon de cette roue dentée triple engrène avec un pignon 25 monté de manière à faire partiellement saillie en dehors du corps d'appareil 1, ainsi que le montre particulièrement la figure 3. Le troisième pignon de cette roue dentée triple engrène avec le pignon 26 faisant partie de l'ensemble 11.

Ce pignon 26 forme corps avec la came 27 de l'ensemble 11, cet ensemble 11 étant monté sur le châssis 5 au moyen de l'axe 28 que comprend cet ensemble 11. Cet axe 28 est chassé dans des paliers que comprend le châssis 5 et est engagé au travers de l'ensemble 11.

La came 27 présente une forme cylindrique et comprend une gorge 30 inclinée par rapport à l'axe de révolution de cette came 27.

Ainsi que cela se comprend en référence aux figures, la partie d'extrémité libre 21 du sous-ensemble 16 s'engage dans la gorge 30 lors de l'adaptation de la tête 2 incluant ce sous-ensemble 16 sur le corps d'appareil 1, le sous-ensemble 16 venant ainsi en accouplement avec la came 27. Cette dernière forme un organe de transformation du mouvement rotatif généré par le moteur 7 en un mouvement de translation alternatif du sous-ensemble 16.

Inversement, cette partie d'extrémité libre 21 est désaccouplée d'avec la came 27 lors du retrait de la tête 2 par rapport au corps d'appareil 1.

Lors de la mise en place de la tête 2 sur le corps d'appareil 1, la forme ogivale de la partie d'extrémité libre 21 permet, grâce à la pointe qu'elle présente, une insertion de la partie 21 dans la gorge 30 quelles que soient la position angulaire de la came 27 et la position en translation du sous-ensemble 16, et permet ainsi d'assurer l'accouplement de cette partie 21 et de cette came 27 quelles que soient les positions relatives de cette partie 21 et de cette came 27. Lesdits bords latéraux convexes de cette partie 21 permettent de limiter les frottements de la partie 21 avec les flancs de la came 27 délimitant latéralement la gorge 30.

Les autres têtes 2 comportant des sous-ensembles mobiles selon des mouvements de translation alternatif présentent des parties d'extrémités libres 21 de mêmes formes que celle décrite ci-dessus, pouvant par conséquent également s'accoupler avec la came 27 lors de l'adaptation de la tête 2 sur le corps 1.

Chaque tête 2 comportant un sous-ensemble mobile selon un mouvement de rotation comprend un pignon venant en prise avec le pignon 25 lors de l'adaptation de la tête 2 sur le corps d'appareil 1, ce pignon 25 permettant ainsi l'entraînement en rotation de ce sous-ensemble.

Comme cela apparaît de ce qui précède, l'invention fournit un appareil de type rasoir, tondeuse ou épilateur, à têtes amovibles 2, comprenant une transmission qui inclut un organe 27 de transformation de mouvement, d'une part, et un organe 25 de transmission de mouvement de rotation, d'autre part, indépendants l'un de l'autre, et comprenant des têtes amovibles 2 pourvues de moyens d'accouplement respectifs, venant en prise avec l'un ou l'autre de ces organes 27, 25.

Les têtes 2 de l'appareil selon l'invention n'incluent ainsi aucun organe de transformation de mouvement à l'intérieur d'elles et ont par conséquent pour avantage déterminant d'avoir des structures relativement simples.

Il va de soi que l'invention n'est pas limitée à la forme de réalisation décrite ci-dessus à titre d'exemple mais qu'elle s'étend à toutes les formes de réalisation couvertes par les revendications ci-annexées.

## Revendications

1. Appareil de type rasoir, tondeuse, épilateur et/ou "exfoliateur", comprenant un corps d'appareil (1) incluant un moteur (7) et une transmission (9, 11 , 25), et comprenant plusieurs têtes amovibles (2) adaptables sur ce corps, au moins une des têtes amovibles (2) comprenant un sous-ensemble (16) mobile selon un mouvement de translation alternatif et au moins une autre des têtes amovibles comprenant un sous-ensemble mobile selon un mouvement de rotation ;
- la transmission (9, 11 , 25) comprenant un organe (27) de transformation de mouvement, entraîné en rotation par l'arbre de sortie du moteur (7) ou par un pignon (26) que comprend la transmission (9, 11 , 25), le sous-ensemble (16) mobile selon un mouvement de translation alternatif comprenant un moyen d'accouplement (20, 21) pouvant être accouplé avec ledit organe (27) de transformation de mouvement lors de l'adaptation de la tête (2) incluant ce sous-ensemble (16) sur ledit corps d'appareil (1), et pouvant être désaccouplé d'avec cet organe (27) de transformation de mouvement lors du retrait de cette tête (2) par rapport audit corps d'appareil (1) ; et
- la transmission (9, 11 , 25) comprenant un organe (25) de transmission de mouvement de rotation, indépendant dudit organe (27) de transformation de mouvement, le sous-ensemble mobile selon un mouvement de rotation comprenant un moyen d'accouplement pouvant être accouplé avec ledit organe (25) de transmission de mouvement de rotation lors de l'adaptation de la tête incluant ce sous-ensemble sur le corps d'appareil (1), et pouvant être désaccouplé d'avec cet organe de transmission (25) lors du retrait de cette tête par rapport audit corps d'appareil (1).
appareil **caractérisé en ce que** :
- ledit organe de transformation de mouvement est formé par une came (27) de forme cylindrique, comprenant une gorge (30) inclinée par rapport à l'axe de révolution de cette came (27) ;
- ledit moyen d'accouplement du sous-ensemble (16) mobile selon un mouvement de translation alternatif est formé par une patte (20) solidaire du sous-ensemble mobile (16) et comprenant une partie d'extrémité libre (21) destinée à être engagée dans la gorge (30) de ladite came (27) lors de l'adaptation de la tête (2) sur le corps d'appareil (1).

2. Appareil selon la revendication 1, **caractérisé en ce que** ladite tête (2) incluant un sous-ensemble (16) mobile selon un mouvement de translation alternatif est un rasoir, une tondeuse ou un "exfoliateur".

3. Appareil selon la revendication 1, **caractérisé en ce que** ladite tête incluant un sous-ensemble mobile selon un mouvement de rotation est un épilateur.

4. Appareil selon l'une des revendications 1 à 3, **caractérisé en ce que** ledit organe de transmission de mouvement de rotation est formé par un pignon (25) faisant saillie du corps d'appareil (1).

5. Appareil selon l'une des revendications 1 ou 4, **caractérisé en ce que** ladite partie d'extrémité libre (21) de ladite patte (20) présente une forme ogivale, c'est-à-dire une extrémité libre pointue et des bords latéraux convexes.

6. Appareil selon l'une des revendications 1, 4 ou 5, **caractérisé en ce que** la came (27) forme corps avec le pignon (26) qui permet l'entraînement de cette came (27) en rotation.

7. Appareil selon la revendication 6, **caractérisé en ce que** la came (27) est montée sur le corps d'appareil (1) au moyen d'un axe (28) chassé dans des paliers que comprend le corps d'appareil (1) et au travers de l'ensemble (11) formé par cette came (27) et ce pignon (26).

## Patentansprüche

1. Gerät mit Rasierapparat-, Haarschneide-, Epilier- und/oder Peelingfunktion mit einem Gerätegehäuse (1), das einen Motor (7) und ein Übersetzungsgetriebe (9, 11, 25) umfasst, und mit mehreren abnehmbaren Kopfstücken (2), die an diesem Gehäuse montiert werden können, wobei mindestens eines dieser abnehmbaren Kopfstücke (2) eine schwenkbar gelagerte Komponente (16) und mindestens ein weiteres der abnehmbaren Kopfstücke eine drehbar gelagerte Komponente umfasst;
- wobei das Übersetzungsgetriebe (9, 11, 25) eine Bewegungsumsetzungseinrichtung (27) aufweist, die über die Abtriebswelle des Motors (7) oder ein Zahnritzel (26) in Drehung versetzt wird, das Teil des Übersetzungsgetriebes (9, 11, 25) ist, wobei die schwenkbar gelagerte Komponente (16) mit einem Eingriffmittel (20, 21) versehen ist, das bei Aufsetzen des mit dieser Komponente (16) ausgestatteten Kopfstücks (2) auf dem Gerätegehäuse (1) mit der genannten Bewegungsumsetzungseinrichtung (27) verkoppelt und bei Abnehmen dieses Kopfstücks (2) vom Gerätegehäuse (1) von der Bewegungsumsetzungseinrichtung (27) abgekoppelt werden kann, und
- wobei das Übersetzungsgetriebe (9, 11, 25) eine Drehbewegungsumsetzungseinrichtung (25) aufweist, die von der genannten Bewegungsumsetzungseinrichtung (27) unabhängig ist, wobei die drehbar gelagerte Komponente mit einem Eingriffmittel versehen ist, das bei Aufsetzen des mit dieser Komponente ausgestatteten Kopfstücks auf dem Gerätegehäuse (1) mit der genannten Drehbewegungsumsetzungseinrichtung (25) verkoppelt und bei Abnehmen dieses Kopfstücks vom Gerätegehäuse (1) von der Umsetzungseinrichtung (25) abgekoppelt werden kann,
**dadurch gekennzeichnet,**
- **dass** die Bewegungsumsetzungseinrichtung als zylindrisch geformter Nocken (27) ausgebildet ist, der eine in Richtung der Rotationsachse des Nockens (27) verlaufende Hohlkehle (30) aufweist;
- **dass** das Eingriffmittel der schwenkbar gelagerten Komponente (16) als mit dieser Komponente (16) verbundene Klammer (20) ausgebildet ist und ein freies Ende (21) aufweist, das bei Aufsetzen des Kopfstücks (2) auf dem Gerätegehäuse (1) in die Hohlkehle (30) des Nockens (27) eingesetzt wird.

2. Gerät nach Anspruch 1, **dadurch gekennzeichnet, dass** es sich bei dem mit einer schwenkbar gelagerten Komponente (16) versehenen Kopfstück (2) um einen Rasier - , Haarschneide- oder Peelingaufsatz handelt.

3. Gerät nach Anspruch 1, **dadurch gekennzeichnet, dass** es sich bei dem mit einer drehbar gelagerten Komponente versehenen Kopfstück um einen Epilieraufsatz handelt.

4. Gerät nach einem der Ansprüche 1 bis 3, **dadurch gekennzeichnet, dass** die Drehbewegungsumsetzungseinrichtung als Zahnritzel (25) ausgebildet ist, das aus dem Gerätegehäuse (1) herausragt.

5. Gerät nach einem der Ansprüche 1 oder 4, **dadurch gekennzeichnet, dass** das freie Ende (21) der Klammer (20) spitzbogenförmig ausgeführt ist, d. h. mit einem spitz zulaufenden freien Ende und konvex gewölbten seitlichen Rändern.

6. Gerät nach einem der Ansprüche 1, 4 oder 5, **dadurch gekennzeichnet, dass** der Nocken (27) mit dem Zahnritzel (26) einen Körper bildet, über den der Nocken (27) in Drehung versetzt werden kann.

7. Gerät nach Anspruch 6, **dadurch gekennzeichnet, dass** der Nocken (27) über eine Achse (28) am Gerätegehäuse (1) montiert ist, die in Achslagern gehalten wird, welche Teil des Gerätegehäuses (1) sind, sowie mithilfe einer aus dem Nocken (27) und dem Zahnritzel (26) geformten Einheit (11).

## Claims

1. Appliance of type razor, clipper, depilator and/or "exfoliator", comprising an appliance body (1) including a motor (7) and a transmission (9, 11, 25), and comprising several removable heads (2) adaptable on this body, at least one of the removable heads (2) comprising a subassembly (16) movable according to a reciprocating translational motion and at least another one of the removable heads comprising a subassembly movable according to a rotational motion;
- the transmission (9, 11, 25) comprising a member (27) for transforming motion, driven in rotation by the output shaft of the motor (7) or by a gear (26) which comprises the transmission (9, 11, 25), the subassembly (16) movable according to a reciprocating translational motion comprising a coupling means (20, 21) which can be coupled with said member (27) for transforming motion when adapting the head (2) including the subassembly (16) on said appliance body (1) and which can be uncoupled from this member (27) for transforming motion when removing this head (2) from said appliance body (1);
and
- the transmission (9, 11, 25) comprising a member (25) for transmitting rotational motion, independent from said member (27) for transforming motion, the subassembly movable according to a rotational motion comprising a coupling means which can be coupled with said member (25) for transmitting rotational motion when adapting the head including this subassembly on the appliance body (1) and which can be uncoupled from this member (25) for transmitting motion when removing this head from said appliance body (1).
appliance **characterised in that**:
- said member for transforming motion consists of a cylindrical cam (27), comprising a groove (30) inclined relative to the axis of revolution of this cam (27);
- said coupling means of the subassembly (16) movable according to a reciprocating translational motion consists of a lug (20) attached to the movable subassembly (16) and comprising a free end portion (21) intended to be engaged in the groove (30) of said cam (27) when adapting the head (2) on the appliance body (1).

2. Appliance according claim 1, **characterised in that** said head (2) including a subassembly (16) movable according to a reciprocating translational motion is a razor, a clipper or an "exfoliator".

3. Appliance according to claim 1, **characterised in that** said head including a subassembly movable according to a rotational motion is a depilator.

4. Appliance according to one of claims 1 to 3, **characterised in that** said member for transmitting rotational motion consists of a gear (25) projecting from the appliance body (1).

5. Appliance according to claim 1 or 4, **characterised in that** said free end portion (21) of said lug (20) is bullet-shaped, that is to say a pointed free end and convex lateral edges.

6. Appliance according to one of claims 1, 4 or 5, **characterised in that** the cam (27) is integral with the gear (26) used to rotate this cam (27).

7. Appliance according to claim 6, **characterised in that** the cam (27) is mounted on the appliance body (1) by means of a pin (28) driven in bearings fitted in the appliance body (1) and through the assembly (11) consisting of this cam (27) and this gear (26).
